# EUROPEAN PATENT SPECIFICATION

(11) **EP 0 815 165 B1**
(45) Date of publication and mention of the grant of the patent: **10.10.2001**
(21) Application number: 96912413.0
(22) Date of filing: 08.03.1996
(51) Int. Cl.: C08J 9/14

(54) **AZEOTROPIC COMPOSITIONS OF PERFLUOROHEXANE, OR 1,1,1,4,4,4-HEXAFLUOROBUTANE, OR 1,3-DIOXOLANE AND HYDROCARBONS HAVING 5 OR 6 CARBON ATOMS**
AZEOTROPISCHE ZUSAMMENSETZUNGEN VON PERFLUOROHEXAN, ODER 1,1,1,4,4-HEXAFLUOROBUTAN, ODER 1,3 DIOXOLAN UND KOHLENWASSERSTOFFE MIT 5 ODER 6 KOHLENSTOFFATOMEN
COMPOSITIONS AZEOTROPES DE PERFLUOROHEXANE OU DE 1,1,1,4,4,4-HEXAFLUOROBUTANE OU DE 1,3-DIOXOLANE ET D'HYDROCARBONES COMPORTANT 5 OU 6 ATOMES DE CARBONE

(30) Priority: 24.03.1995 US 410469; 24.03.1995 US 410458; 24.03.1995 US 410608; 24.03.1995 US 410247; 24.03.1995 US 410177
(43) Date of publication of application: 07.01.1998
(73) Proprietor: Bayer Corporation, Pittsburgh, PA 15205-9741 (US)
(72) Inventor: WERNER, Joachim, Bethel Park, PA 15102 (US); KANE, Scott, A., Wellsburg, WV 26070 (US); DOERGE, Herman, P., Pittsburg, PA 15237 (US); BOONSTRA, Eric, F., Oakdale, PA 15071 (US)
(74) Representative: Pettrich, Klaus-Günter, Dr.
(86) International application number: US9603412
(87) International publication number: WO9630439

(56) References cited:
- EP-A- 0 607 969
- DE-A- 4 143 148
- US-A- 5 290 823
- US-A- 5 403 514
- CHEMICAL ABSTRACTS, vol. 123, no. 6, 7 August 1995 Columbus, Ohio, US; abstract no. 58290, XP002007323 & JP,A,07 102 105 (TOHO CHEM IND CO LTD) 18 April 1995

## Description

The present invention relates to novel azeotropic compositions, a process for the production of foams in which these azeotropic compositions are used and to foams produced using these azeotropic compositions.

The use of trichloromonofluoromethane (CFC-11) and other chlorofluorocarbons as blowing agents in the production of urethane foams is well known. These CFC blowing agents are also known to have an adverse effect upon the ozone layer in the atmosphere. The urethane foam industry is therefore investigating methods for producing foams with good physical properties without using CFC blowing agents.

Initially, the most promising alternatives appeared to be hydrogencontaining chlorofluorocarbons (HCFCs). U.S. Patent 4,076,644, for example, discloses the use of 1,1-dichloro-2,2,2-trifluoroethane (HCFC-123) and 1,1-dichloro-1-fluoroethane (HCFC-141b) as blowing agents for the production of polyurethane foams. However, HCFCs also have some ozone-depletion potential. There is therefore mounting pressure to find substitutes for the HCFCs as well as the CFCs.

Alternative blowing agents which are currently considered promising because they contain no ozone-depleting chlorine are fluorocarbons (FCs) and partially fluorinated hydrocarbons (HFCs). The use of 1,1,1,4,4,4-hexafluorobutane as a blowing agent is disclosed in Lamberts, "1,1,1,4,4,4-hexafluorobutane, a New Non-Ozone-Depleting Blowing Agent for Rigid PUR Foams", Polyurethanes World Congress 1991 (September 24-26), pages 734-739. U.S. Patent 4,972,002 teaches that low boiling fluorinated compounds such as fluorinated hydrocarbons, perfluorinated hydrocarbons, sulfur hexafluoride and mixtures thereof are useful as blowing agents for the production of polyisocyanate polyaddition foam products.

U.S. Patent 4,898,893 teaches that a blend of a liquid hydrocarbon and halogenated hydrocarbon is useful as a blowing agent for the production of isocyanurate foams.

The use of mixtures of a chlorofluorocarbon having a boiling point between 74 and 120°F and an alkyl alkanoate having a molecular weight of no more than 88 as a blowing agent for foams is disclosed in U.S. Patent 4,960,804. HCFC-123 and HCFC-141b are among the chlorofluorocarbons disclosed therein.

U.S. Patent 5,035,833 discloses the use of a mixture of dichlorotrifluoroethane and at least one paraffin having 5 or 6 carbon atoms as blowing agents useful for the production of rigid polyurethane foams.

U.S. Patent 5,096,933 discloses a process for the production of rigid polyurethane foams in which cyclopentane, cyclohexane or cyclopentane and cyclohexane, optionally in combination with a low boiling compound (i.e., boiling point less than 35°C) having no more than 4 carbon atoms which is homogeneously miscible with cyclopentane and/or cyclohexane is used.

U.S. Patent 5,290,823 teaches the production of rigid polyurethane foams using a blowing agent mixture which includes highly fluorinated or perfluorinated organic compounds, cyclopentane and optionally other aliphatic or cycloaliphatic hydrocarbons having 4 to 8 carbon atoms.

U.S. Patent 5,318,996 discloses the production of rigid polyurethane foams with a mixture of blowing agents which mixture includes water, HCFC-22 or HCFC-141b and a perfluorinated hydrocarbon having from 4 to 8 carbon atoms.

Mixtures and azeotropes of HCFCs and various compounds and azeotropes of organic compounds which may be used in combination with HCFCs have also been described in the prior art as being useful blowing agents for the production of foams.

U.S. Patent 4,828,751 teaches solvent compositions for cleaning silicon wafers which solvent compositions include a perhalogenated haloalkylhydrocarbon containing from 5 to 12 carbon atoms and a partially fluorinated alcohol.

U.S. Patent 4,900,365, for example, teaches that azeotropes of a dichlorotrifluoroethane and isopentane are useful in the production of polyurethane foams.

U.S. Patent 5,106,527 discloses the use of azeotropes of 2-methyl butane and 1,1-dichloro-1-fluoroethane as blowing agents for the production of rigid, closed cell foams.

The azeotropic mixtures taught in U.S. Patent 5,166,182 must have boiling points below 50°C. These azeotropic mixtures are formed from organic compounds having surface active properties that enable the blended azeotropic mixture to become miscible with polymer resins. Examples of the organic compounds described as being useful in the production of such azeotropes include: n-pentane, acetone, methyl alcohol, methyl formate, ethyl formate, ethyl alcohol, 2-methyl butane, nitromethane, cyclopentane, 2,3-dimethyl butane, 2,2-dimethyl butane and dimethyl sulfide. These azeotropes may be used in combination with fluorocarbons but an azeotrope in which a fluorocarbon is one of the components is not taught or suggested.

U.S. Patent 5,227,088 discloses azeotrope-like compositions which are made up of 1-chloro-3,3,3-trifluoropropane and a hydrocarbon containing five or six carbon atoms.

U.S. Patent 5,283,003 discloses a blowing agent which is made up of at least one five-carbon member hydrocarbon, a chlorinated alkane and methyl formate. Methylene chloride is the preferred chlorinated alkane.

Azeotropic mixtures in which HCFCs are included are also known to be useful as cleaning solvents. U.S. Patent 4,055,507, for example, discloses an azeotropic mixture of 1,2-dichloro-1,1-difluoroethane and 3-methyl pentane which is taught to be useful as such a solvent. U.S. Patent 5,302,212 teaches that an azeotrope of (perfluoroalkyl) ethylene and methanol may be used to clean a solid surface. Japanese 1,141,995 discloses an azeotropic mixture of 67 to 87% by weight of HCFC-123 and 13 to 33% by weight of 2-methyl butane which is useful as a cleaning solvent. Japanese 1,141,996 discloses an azeotropic mixture of HCFC-141b and n-pentane or 2-methyl butane or 2,2-dimethyl butane which is also taught to be useful as a cleaning solvent.

The use of azeotropes formed from perfluorohexane, or 1,1,1,4,4,4-hexafluorobutane or 1,3-dioxolane and a hydrocarbon having 5 or 6 carbon atoms as a blowing agent or a cleaning solvent has not, however, been described in the prior art.

### SUMMARY OF THE INVENTION

It is an object of the present invention to provide novel azeotropic compositions.

It is a further object of the present invention to provide an azeotropic composition which contains no halogen, particularly, no chlorine and therefore has an ozone depletion potential of zero.

It is also an object of the present invention to provide a process for the production of urethane foams in which no halogen-containing, particularly, no chlorine-containing blowing agent is employed.

It is another object of the present invention to provide polyurethane foams having good physical properties which foams are produced without the use of a halogen-containing, particularly, chlorine-containing, blowing agent.

These and other objects which will be apparent to those skilled in the art are accomplished with the azeotropic compositions of the present invention. These azeotropic compositions are made up of (a) one compound selected from the following: (1) from 26 to 88% by weight perfluorohexane or (2) from 4 to 50% by weight 1,3-dioxolane or (3) from 64 to 87% by weight 1,1,1,4,4,4-hexafluorobutane and (b) one compound selected from the following: (1) 20 to 74% by weight 2-methyl butane, or (2) 13 to 53% by weight n-pentane, or (3) from 75 to 96% by weight cyclopentane, or (4) from 13 to 77% by weight 3-methyl pentane, or (5) from 12 to 69% by weight n-hexane, in which the sum of the weight percent of (a) and weight percent of (b) is 100 percent.

These azeotropic compositions are included in a foam-forming mixture which includes an isocyanate and isocyanate-reactive material. The foams made with these azeotropic compositions are characterized by good physical properties.

### BRIEF DESCRIPTION OF THE DRAWINGS

Figure 1 is a graph showing a plot of the mole fraction of 2-methyl butane in the vapor phase versus the mole fraction of 2-methyl butane in the liquid phase of varying mixtures of 2-methyl butane and perfluorohexane refluxing at steady state at one atmosphere.
Figure 2 is a graph showing a plot of the mole fraction of n-pentane in the vapor phase versus the mole fraction of n-pentane in the liquid phase of varying mixtures of n-pentane and perfluorohexane refluxing at steady state at one atmosphere.
Figure 3 is a graph showing a plot of the mole fraction of 2-methyl butane (i-pentane) in the vapor phase versus the mole fraction of 2-methyl butane in the liquid phase of varying mixtures of 2-methyl butane and 1,1,1,4,4,4-hexafluorobutane refluxing at steady state at one atmosphere.
Figure 4 is a graph showing a plot of the mole fraction of cyclopentane in the vapor phase versus the mole fraction of cyclopentane in the liquid phase of a mixture of cyclopentane and 1,3-dioxolane refluxing at steady state at one atmosphere.
Figure 5 is a graph showing a plot of the mole fraction of 2-methyl pentane in the vapor phase versus the mole fraction of 2-methyl pentane in the liquid phase of a mixture of 2-methyl pentane and 1,3-dioxolane refluxing at steady state at one atmosphere.
Figure 6 is a graph showing a plot of the mole fraction of 3-methyl pentane in the vapor phase versus the mole fraction of 3-methyl pentane in the liquid phase of a mixture of 3-methyl pentane and 1,3-dioxolane refluxing at steady state at one atmosphere.
Figure 7 is a graph showing a plot of the mole fraction of n-hexane in the vapor phase versus the mole fraction of n-hexane in the liquid phase of a mixture of n-hexane and 1,3-dioxolane refluxing at a steady state at one atmosphere.
Figure 8 is a graph showing a plot of the mole fraction of 2-methyl pentane in the vapor phase versus the mole fraction of 2-methyl pentane in the liquid phase of a mixture of 2-methyl pentane and perfluorohexane refluxing at steady state at one atmosphere.
Figure 9 is a graph showing a plot of the mole fraction of 3-methyl pentane in the vapor phase versus the mole fraction of 3-methyl pentane in the liquid phase of a mixture of 3-methyl pentane and perfluorohexane refluxing at steady state at one atmosphere.
Figure 10 is a graph showing a plot of the mole fraction of n-hexane in the vapor phase versus the mole fraction of n-hexane in the liquid phase of a mixture of n-hexane and perfluorohexane refluxing at steady state at one atmosphere.
Figure 11 is a graph showing a plot of the mole fraction of n-pentane in the vapor phase versus the mole fraction of n-pentane in the liquid phase of various mixtures of n-pentane and 1,1,1,4,4,4-hexafluorobutane refluxing at steady state at one atmosphere.

### DETAILED DESCRIPTION OF THE INVENTION

The present invention relates to an azeotropic composition which is particularly useful for the production of rigid foams. This azeotropic composition may also be used for solvent cleaning applications.

The azeotropes of the present invention are generally made up of two components. The first component is one compound selected from perfluorohexane present in an amount of from 26 to 88% by weight (based on total weight of azeotrope), 1,3-dioxolane in an amount of from 4 to 50% by weight (based on total weight of azeotrope) and 1,1,1,4,4,4-hexafluorobutane in an amount of from 64 to 87% by weight (based on total weight of azeotrope). The second component is one compound selected from 2-methyl butane in an amount of from 20 to 74% by weight (based on total weight of azeotrope), n-pentane in an amount of from 13 to 53% by weight (based on total weight of azeotrope), cyclopentane in an amount of from 75 to 96% by weight (based on total azeotrope), 3-methyl pentane in an amount of from 13 to 77% by weight (based on total weight of azeotrope) and n-hexane in an amount of from 12 to 69% by weight (based on total weight of azeotrope). The sum of the weight percents of the first and second components is 100%.

Among the preferred azeotrope-like compositions of the present invention are those which consist essentially of (a) (1) from 26% to 72% by weight of perfluorohexane (based on the total weight of the azeotropic composition) and (b) one compound selected from (1) 36 to 74% by weight 2-methyl butane (based on the total weight of the azeotropic composition) or (2) 28 to 53% by weight n-pentane (based on the total weight of the azeotropic composition). The sum of the weight percent of (a) plus the weight percent of (b) is 100 weight percent.

Other preferred azeotrope-like compositions include those consisting essentially of (a) (3) from 64 to 80% by weight (44 to 63 mol%) of 1,1,1,4,4,4-hexafluorobutane (based on the total weight of the azeotropic composition) and (b) (1) from 20 to 36% by weight (37 to 56 mol%) of 2-methyl butane (also known as isopentane) (based on the total weight of the azeotropic composition).

Another group of preferred azeotrope-like compositions are those consisting essentially of (a) (2) from 4 to 50% by weight of 1,3-dioxolane (based on the total weight of the azeotropic composition) and (b) one compound selected from (3) from 75 to 96% by weight cyclopentane (based on the total weight of the azeotropic composition) (i.e., from about 76 to about 96 mole%) or (4) from 59 to 77% by weight 3-methyl pentane (based on the total weight of the azeotropic composition) (i.e., from about 55 to about 74 mole%) or (5) from 50 to 69% by weight n-hexane (based on the total weight of the azeotropic composition) (i.e., from about 46 to about 66 mole%). The sum of the weight percent of (a) plus the weight percent of (b) is 100 weight percent.

Also preferred are those azeotrope-like compositions consisting essentially of (a) (1) from 73% to 88% by weight of perfluorohexane (based on the total weight of the azeotropic composition) and (b) one compound selected from (4) 13 to 25% by weight 3-methyl pentane (based on the total weight of the azeotropic composition) (i.e., from about 37 to about 57 mole %) or (5) 12 to 23% by weight n-hexane (based on the total weight of the azeotropic composition) (i.e., from about 35 to about 54 mole %). The sum of the weight percent of (a) plus the weight percent of (b) is 100 weight percent.

Other preferred azeotrope-like compositions consist essentially of (a) (3) from 73 to 87% by weight of 1,1,1,4,4,4-hexafluorobutane (based on the total weight of the azeotropic composition) (i.e., from about 54 to about 74 mole %) and (b) (2) from 13 to 27% by weight of n-pentane (based on the total weight of the azeotropic composition) (i.e., from about 26 to about 46 mole %).

The compounds which are essential to the compositions of the present invention are perfluorohexane (boiling point = 59°C), 1,3-dioxolane (boiling point = 75°C), 1,1,1,4,4,4-hexafluorobutane (boiling point = 24.6°C), 2-methyl butane (boiling point = 28.0°C), n-pentane (boiling point = 35.5°C), cyclopentane (boiling point = 49°C), 2-methyl pentane (boiling point = 62°C), 3-methyl pentane (boiling point = 64°C), and n-hexane (boiling point = 69°C). All of these compounds are known to those in the art and are commercially available. These compounds may be used in the compositions of the present invention at their normal commercial purity (i.e., at least 95%).

1,1,1,4,4,4-hexafluorobutane is also known by those skilled in the art as R-356 or HFC 356 mffm. A process for producing 1,1,1,4,4,4-hexafluorobutane is disclosed in U.S. Patent 5,315,047.

Any of the compositions made up of (a)(1) from 26 to 72% by weight perfluorohexane and (b) only one compound selected from (1) 36 to 74% by weight 2-methyl butane or (2) 28 to 53% by weight n-pentane in which the sum of the weight percent of (a) plus the weight percent of (b) is 100 weight percent is azeotropic in nature in that compositions within these ranges exhibit a substantially constant boiling point. Because they have such a substantially constant boiling point, the mixture does not tend to fractionate to any great extent upon evaporation.

The compositions made up of (a)(3) from 64 to 80% by weight 1,1,1,4,4,4-hexafluorobutane and (b)(1) from 20 to 36% by weight 2-methyl butane are azeotropic in nature in that compositions within these ranges exhibit a substantially constant boiling point. Because they have such a substantially constant boiling point (15.8°C at one atmosphere), the mixture does not tend to fractionate to any great extent upon evaporation.

Any of the compositions made up of (a)(2) from 4 to 50% by weight 1,3-dioxolane and (b) only one compound selected from (3) from 75 to 96% by weight cyclopentane or (4) from 59 to 77% by weight 3-methyl pentane or (5) from 50 to 69% by weight n-hexane in which the sum of the weight percent of (a) plus the weight percent of (b) is 100 weight percent is azeotropic in nature in that compositions within these ranges exhibit a substantially constant boiling point.

Any of the compositions made up of (a)(1) from 73 to 88% by weight perfluorohexane and (b) only one compound selected from (4) 13 to 25% by weight 3-methyl pentane or (5) 12 to 23% by weight n-hexane in which the sum of weight percent of (a) plus weight percent of (b) is 100 weight percent is azeotropic in nature in that compositions within these ranges exhibit a substantially constant boiling point.

The compositions made up of (a)(3) from 73 to 87% by weight 1,1,1,4,4,4-hexafluorobutane and (b)(2) from 13 to 27% by weight n-pentane are azeotropic in nature in that compositions within these ranges exhibit a substantially constant boiling point.

Because they have such a substantially constant boiling point, the mixtures do not tend to fractionate to any great extent upon evaporation. After evaporation, only a small difference exists between the composition of the vapor phase and the initial liquid phase. This difference is so small that the compositions of the vapor and liquid phases are considered to be substantially identical. Therefore, any mixture within the above-noted ranges exhibits properties which are characteristic of a true binary azeotrope.

Azeotropic compositions consisting essentially of (a)(1) from 40 to 68% by weight perfluorohexane and (b) only one compound selected from (1) 43 to 60% by weight 2-methyl butane or (2) 32 to 44% n-pentane in which the sum of the weight percent of (a) plus the weight percent of (b) is 100 are particularly preferred azeotropic compositions. The azeotropic compositions made up of perfluorohexane and 2-methyl butane consist essentially of from 26 to 64% (preferably from 40 to 57%) by weight perfluorohexane and from 36 to 74% (preferably from 43 to 60%) by weight 2-methyl butane. The composition consisting essentially of 49% by weight perfluorohexane and 51% by weight 2-methyl butane has been established, within the accuracy of the calibration procedure described below, as the true binary azeotrope with a boiling point of 25.0°C.

The azeotropic compositions made up of perfluorohexane and n-pentane consist essentially of from 47 to 72% (preferably from 56 to 68%) by weight perfluorohexane and from 28 to 53% (preferably from 32 to 44%) by weight n-pentane. The composition consisting essentially of 62% by weight perfluorohexane and 38% by weight n-pentane has been established, within the accuracy of the calibration procedure described below, as the true binary azeotrope with a boiling point of 30.3°C.

The azeotropic compositions consisting essentially of from 68 to 76% by weight 1,1,1,4,4,4-hexafluorobutane and from 24 to 32% by weight 2-methyl butane are particularly preferred azeotropic compositions. The composition consisting essentially of 72% by weight 1,1,1,4,4,4-hexafluorobutane and 28% 2-methyl butane has been established, within the accuracy of the calibration procedure described below, as the true binary azeotrope with a boiling point of about 15.8°C.

Azeotropic compositions consisting essentially of (a)(2) from 10 to 45% by weight 1,3-dioxolane and only one compound selected from (b) (3) from 80 to 90% by weight cyclopentane or (4) from 63 to 73% 3-methyl pentane or (5) from 55 to 64% by weight n-hexane in which the sum of weight percent of (a) plus weight percent of (b) is 100 are also particularly preferred azeotropic compositions.

The azeotropic compositions made up of 1,3-dioxolane and cyclopentane consist essentially of from 4 to 25% (preferably from 10 to 20%) by weight 1,3-dioxolane and from 75 to 96% (preferably from 80 to 90%) by weight cyclopentane. The composition consisting essentially of 14% by weight 1,3-dioxolane and 86% by weight cyclopentane has been established, within the accuracy of the calibration procedure described below, as the true binary azeotrope with a boiling point of about 48.5°C.

The azeotropic compositions made up of 1,3-dioxolane and 3-methyl pentane consist essentially of from 33 to 41% (preferably from 27 to 37%) by weight 1,3-dioxolane and from 59 to 77% (preferably from 63 to 73%) by weight 3-methyl pentane. The composition consisting essentially of 32% by weight 1,3-dioxolane and 68% by weight 3-methyl pentane has been established, within the accuracy of the calibration procedure described below, as the true binary azeotrope with a boiling point of 58.6°C.

The azeotropic compositions made up of 1,3-dioxolane and n-hexane consist essentially of from 31 to 50% (preferably from 36 to 45%) by weight 1,3-dioxolane and from 50 to 69% (preferably from 55 to 64%) by weight n-hexane. The composition consisting essentially of 40% by weight 1,3-dioxolane and 60% by weight n-hexane has been established, within the accuracy of the calibration procedure described below, as the true binary azeotrope with a boiling point of 62.8°C.

Azeotropic compositions consisting essentially of (a)(1) from 77 to 86% by weight perfluorohexane and (b) only one compound selected from (4) 16 to 22% 3-methyl pentane or (5) 14 to 20% by weight n-hexane in which the sum of weight percent of (a) plus weight percent of (b) is 100 are also preferred azeotropic compositions.

The azeotropic compositions made up of perfluorohexane and 3-methyl pentane consist essentially of from 75 to 87% (preferably from 78 to 84%) by weight perfluorohexane and from 13 to 25% (preferably from 16 to 22%) by weight 3-methyl pentane. The composition consisting essentially of 82% by weight perfluorohexane and 18% by weight 3-methyl pentane has been established, within the accuracy of the calibration procedure described below, as the true binary azeotrope with a boiling point of 46.4°C.

The azeotropic compositions made up of perfluorohexane and n-hexane consist essentially of from 77 to 88% (preferably from 80 to 86%) by weight perfluorohexane and from 12 to 23% (preferably from 14 to 20%) by weight n-hexane. The composition consisting essentially of 83% by weight perfluorohexane and 17% by weight n-hexane has been established, within the accuracy of the calibration procedure described below, as the true binary azeotrope with a boiling point of 48.0°C.

The azeotropic compositions consisting essentially of from 77 to 84% by weight 1,1,1,4,4,4-hexafluorobutane and from 16 to 23% by weight n-pentane are particularly preferred azeotropic compositions. The composition consisting essentially of 80% by weight 1,1,1,4,4,4-hexafluorobutane and 20% by weight n-pentane has been established, within the accuracy of the calibration procedure described below, as the true binary azeotrope with a boiling point of about 18.5°C.

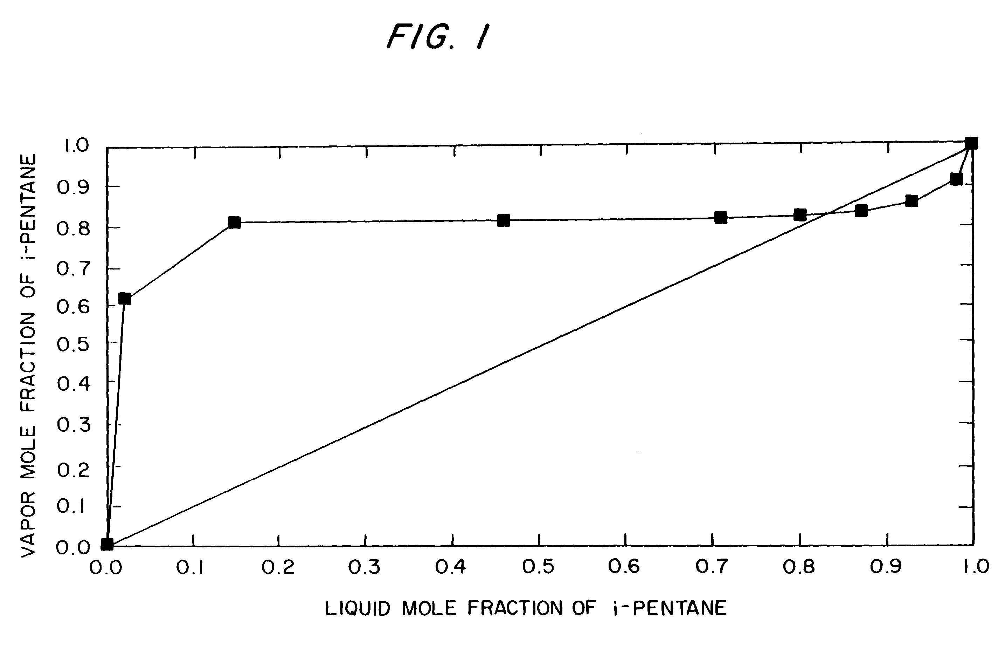

Figure 1 shows a graph on which the mole fraction of 2-methyl butane in the vapor phase is plotted against the mole fraction of 2-methyl butane in the liquid phase of varying mixtures of 2-methyl butane and perfluorohexane refluxing at steady state and at 1 atmosphere. These mole fractions were obtained by gas chromatography and were adjusted to be quantitative by using a calibration curve as is described more fully below. The point at which the mole fraction curve crosses the line with a slope of 1 and intercept 0 is, by definition, the true binary azeotropic composition.

The calibration curve used to calibrate the gas chromatographic results was generated as follows. A series of blends of 2-methyl butane and perfluorohexane were prepared with from 0 to 100 mole percent of 2-methyl butane in 10% increments. The mole percent of perfluorohexane in each blend was the difference between 100 mole percent and the mole percent of 2-methyl butane. First, each blend was injected into a Gas Chromatograph ("GC") to establish a correlation between relative peak areas versus actual molar concentrations. This was done by making duplicate samples of each blend and measuring each of the samples twice. This data was used to establish the calibration curve and a 95% confidence interval which was used to establish the range of error for the azeotropic compositions.

The relative molar amounts of perfluorohexane and 2-methyl butane necessary to form an azeotropic composition were then determined by a two step process. In the first step, 2-methyl butane was charged to a reactor. Subsequently, perfluorohexane was added to the reactor in increments indicated by the data points in the graphs. After each addition of perfluorohexane, the contents of the reactor were allowed to reflux for 10-15 minutes with the reflux condenser at 0°C and open to the atmosphere through a drying tube. After steady state was achieved, samples of the liquid and vapor were taken through sample ports. The temperature of the liquid in the reactor was measured and the vapor temperature was measured at a point between the reactor and the condenser. Duplicate samples were injected into the GC and the relative peak areas were recorded. These relative peak areas were converted to mole fractions by using the calibration curve.

In the second step, perfluorohexane was charged to a reactor. Subsequently, 2-methyl butane was added in increments indicated by the data points in the graphs. The contents of the reactor were then heated and samples were taken and analyzed in the same manner described above in the first step. The data was plotted with the resultant graph being shown in Figure 1.

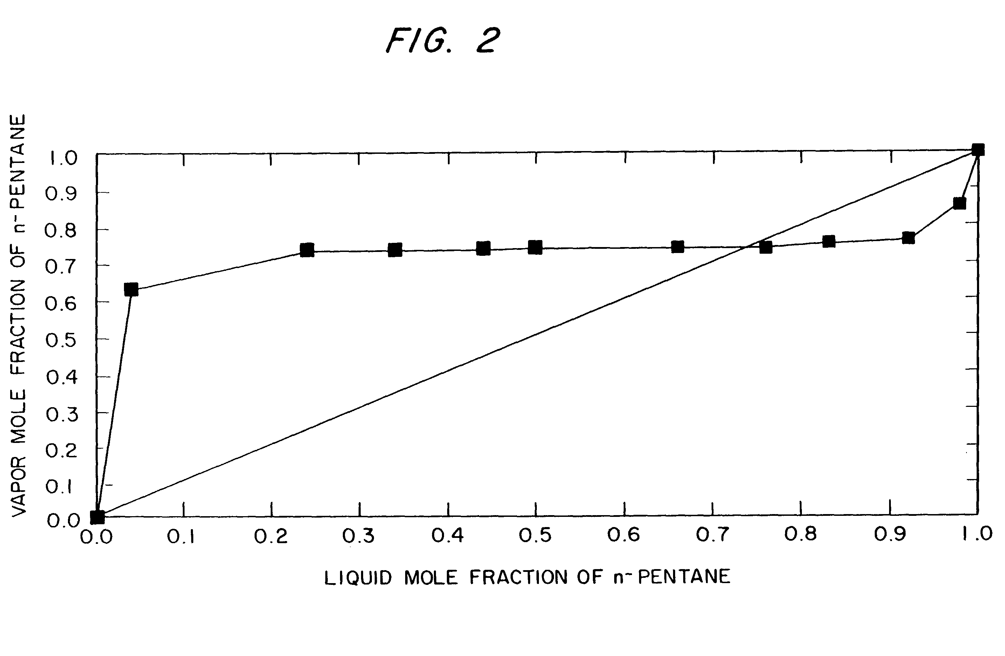

The graph shown in Figure 2 was generated in the same manner as Figure 1 using blends of perfluorohexane and n-pentane.

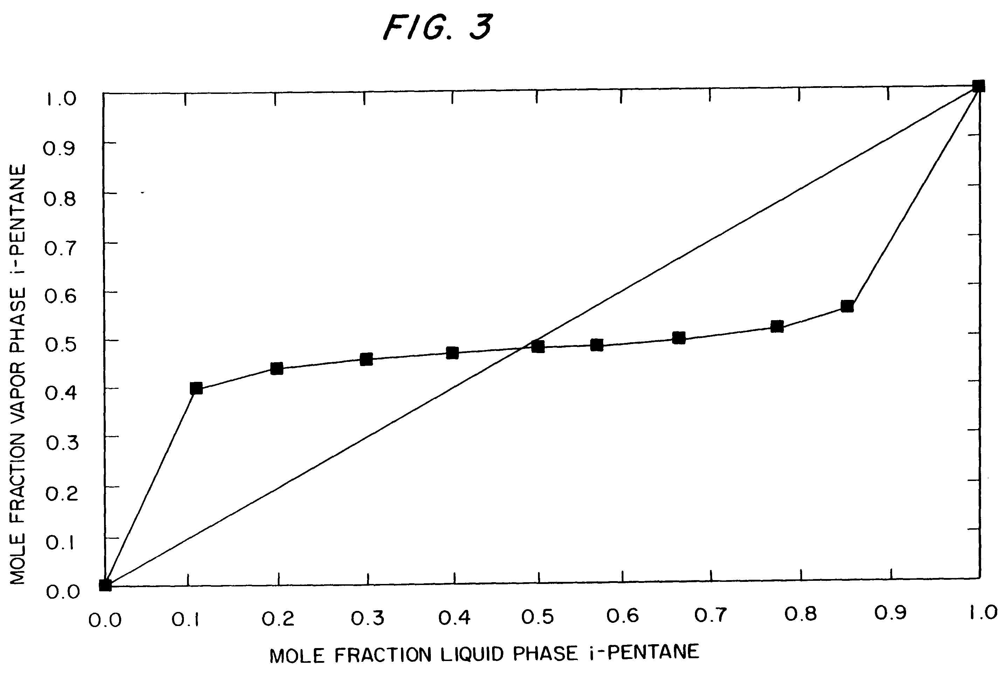

Figure 3 shows a graph on which the mole fraction of 2-methyl butane in the vapor phase is plotted against the mole fraction of 2-methyl butane in the liquid phase of a mixture of 2-methyl butane and 1,1,1,4,4,4-hexafluorobutane refluxing at steady state. These mole fractions were obtained by gas chromatography and were adjusted to be quantitative by using a calibration curve as is described more fully below. The point at which the mole fraction curve crosses the line with a slope of 1 and intercept 0 is, by definition, the true binary azeotropic composition.

The calibration curve used to calibrate the gas chromatographic results was generated as follows. A series of blends of 2-methyl butane with 1,1,1,4,4,4-hexafluorobutane were prepared with from 0 to 100 mole percent of 2-methyl butane in 10% increments. The mole percent of 1,1,1,4,4,4-hexafluorobutane in each blend was the difference between 100 mole percent and the mole percent of 2-methyl butane. First, each blend was injected into a GC to establish a correiation between relative peak areas versus actual molar concentrations-. This was done by making duplicate samples of each blend and measuring each sample twice. This data was used to establish the calibration curve and a 95% confidence interval which was used to establish the range of error for the azeotropic compositions.

The relative molar amounts of 1,1,1,4,4,4-hexafluorobutane and 2-methyl butane necessary to form an azeotropic composition were then determined by a two-step process. In the first step, 2-methyl butane alone was charged to a reactor. Subsequently, 1,1,1,4,4,4-hexafluorobutane was added to the reactor in regular increments indicated by the data points in the graph. After each addition of 1,1,1,4,4,4-hexafluorobutane, the contents of the reactor were allowed to reflux for 10-15 minutes with the reflux condenser at 0°C and open to the atmosphere through a drying tube. After a steady state was achieved, samples of the liquid and vapor were taken through sample ports. The temperature of the liquid in the reactor was measured and the vapor temperature was measured at a point between the reactor and the condenser. Duplicate samples were injected into the GC and the relative peak areas were recorded. These relative peak areas were converted to mole fractions by using the calibration curve.

In the second step, 1,1,1,4,4,4-hexafluorobutane was charged to a reactor. Subsequently, 2-methyl butane was added in increments indicated by the data points in the graph. The contents of the reactor were then heated and samples were taken and analyzed in the same manner as was described above in the first step. The data was plotted with the resultant graph being shown in the Figure 3.

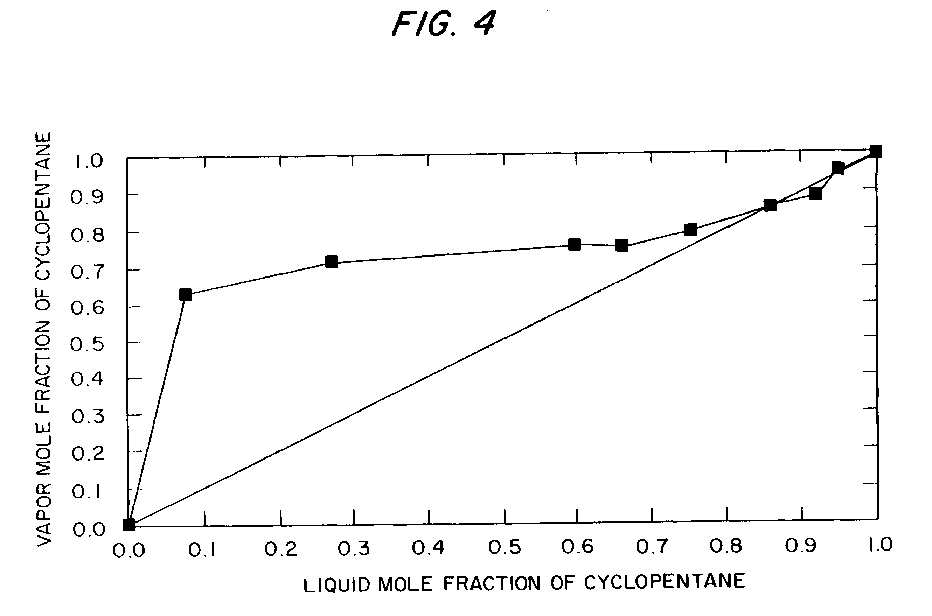

Figure 4 shows a graph on which the mo!e fraction of cyclopentane in the vapor phase is plotted against the mole fraction of cyclopentane in the liquid phase of a mixture of cyclopentane and 1,3-dioxolane refluxing at steady state and at 1 atmosphere. These mole fractions were obtained by gas chromatography and were adjusted to be quantitative by using a calibration curve as is described more fully below. The point at which the mole fraction curve crosses the line with a slope of 1 and intercept 0 is, by definition, the true binary azeotropic composition.

The calibration curve used to calibrate the gas chromatographic results was generated as follows. A series of blends of cyclopentane and 1,3-dioxolane were prepared with from 0 to 100 mole percent cyclopentane in 10% increments. The mole percent of 1,3-dioxolane in each blend was the difference between 100 mole percent and the mole percent of cyclopentane. First, each blend was injected into a GC to establish a correlation between relative peak areas versus actual molar concentrations. This was done by making duplicate samples of each blend and measuring each sample twice. This data was used to establish the calibration curve and a 95% confidence interval which was used to establish the range of error for the azeotropic compositions.

The relative molar amounts of 1,3-dioxolane and cyclopentane necessary to form an azeotropic composition were then determined by a two-step process. In the first step, cyclopentane was charged to a reactor. Subsequently, 1,3-dioxolane was added to the reactor in increments indicated by the data points in the graph. After each addition of 1,3-dioxolane, the contents of the reactor were allowed to reflux for 10-15 minutes with the reflux condenser at 0°C and open to the atmosphere through a drying tube. After steady state was achieved, samples of the liquid and vapor were taken through sample ports. The temperature of the liquid was measured and the temperature of the vapor was measured at a point between the reactor and the condenser. Duplicate samples were injected into the GC and the relative peak areas were recorded. These relative peak areas converted to mole fractions using the calibration curve.

In the second step, 1,3-dioxolane was charged to the reactor. Subsequently, cyclopentane was added to the reactor in increments indicated by the data points in the graph. The contents of the reactor were then heated and samples were taken and analyzed in the same manner as was described above with respect to the first step. The data was plotted with the resultant graph being shown in Figure 1.

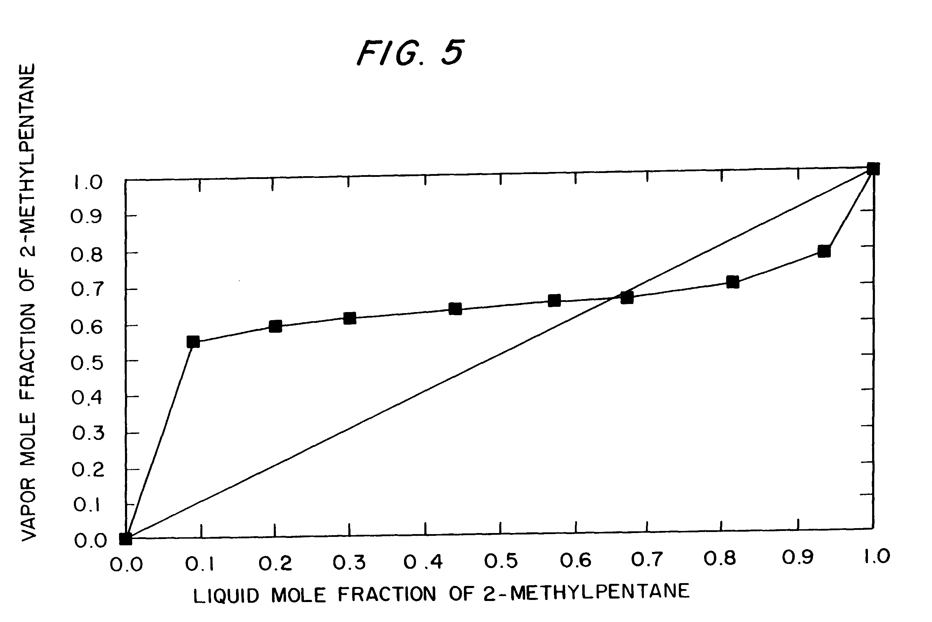

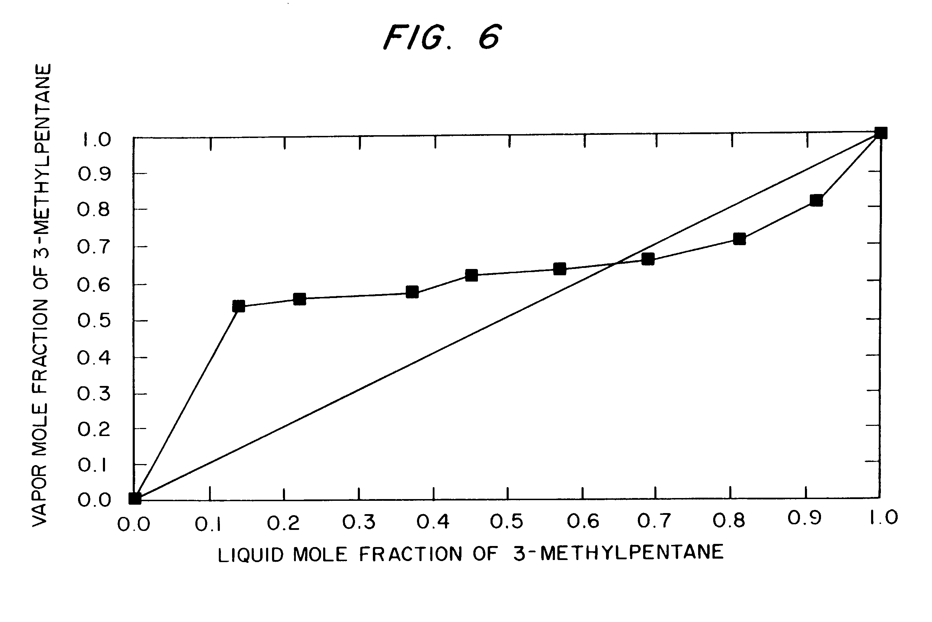

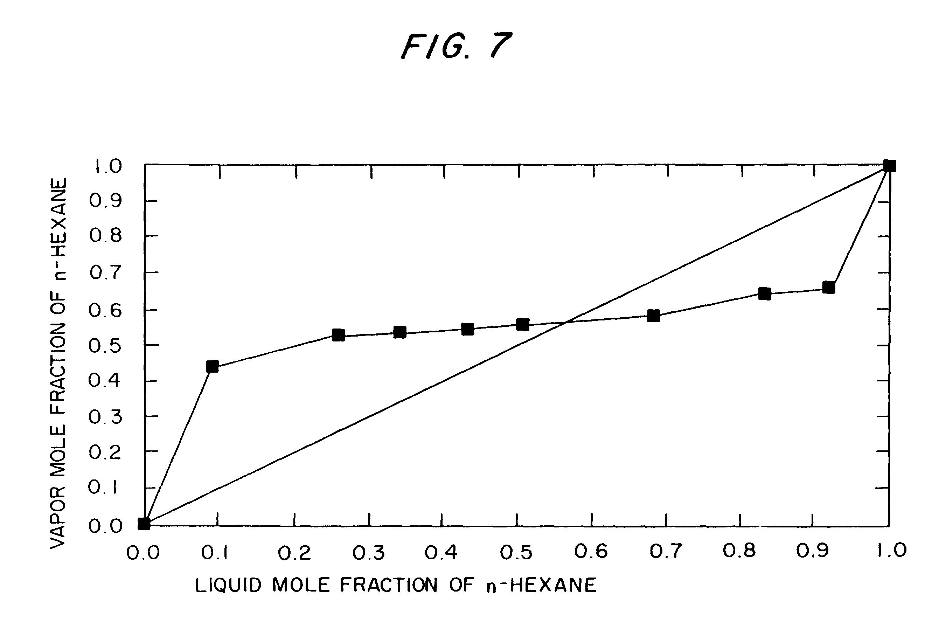

The graphs shown in Figures 5, 6 and 7 were generated in the same manner as Figure 4 using blends of 1,3-dioxolane and 2-methyl pentane (Figure 5), 1,3-dioxolane and 3-methyl pentane (Figure 6) and 1,3-dioxolane and n-hexane (Figure 7).

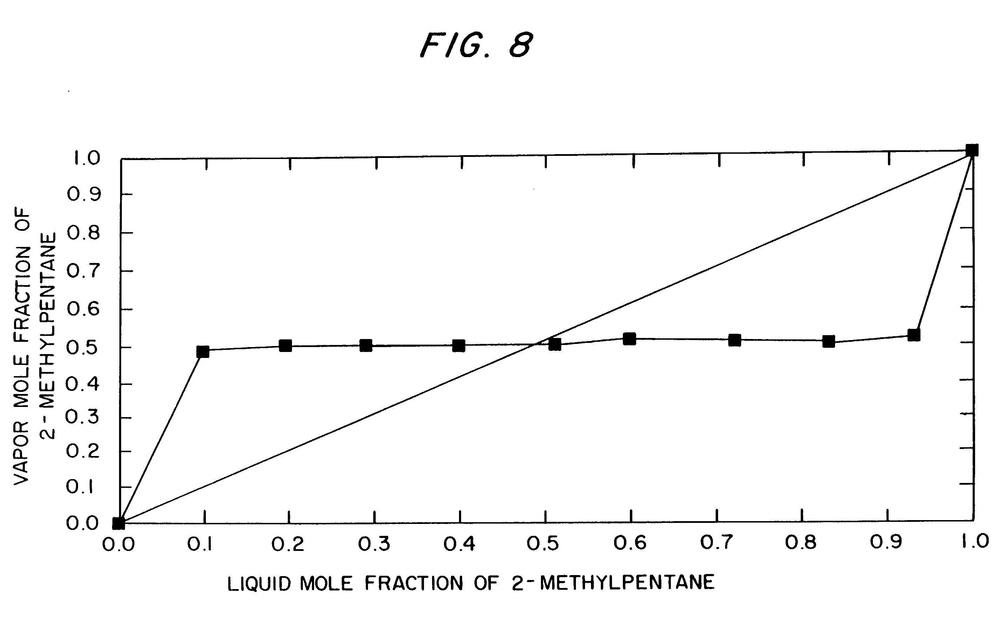

Figure 8 shows a graph on which the mole fraction of 2-methyl pentane in the vapor phase is plotted against the mole fraction of 2-methyl pentane in the liquid phase of varying mixtures of 2-methyl pentane and perfluorohexane at steady state and at one atmosphere. These mole fractions were obtained by gas chromatography and were adjusted to be quantitative by using a calibration curve as is described more fully below. The point at which the mole fraction curve crosses the line with a slope of 1 and intercept 0 is, by definition, the true binary azeotropic composition.

The calibration curve used to calibrate the gas chromatographic results was generated as follows. A series of blends of 2-methyl pentane and perfluorohexane were prepared with from 0 to 100 mole percent of 2-methyl pentane in 10% increments. The mole percent of perfluorohexane in each blend was the difference between 100 mole percent and the mole percent of 2-methyl pentane. First, each blend was injected into a GC to establish a correlation between relative peak areas versus actual molar concentrations. This was done by making duplicate samples of each blend and measuring each sample twice. This data was used to establish the calibration curve and a 95% confidence interval which was used to establish the range of error for the azeotropic compositions.

The relative molar amounts of perfluorohexane and 2-methyl pentane necessary to form an azeotropic composition were then determined by a two step process. In the first step, 2-methyl pentane was charged to a reactor. Subsequently, perfluorohexane was added to the reactor in regular increments indicated by the data points in the graphs. After each addition, the contents of the reactor were allowed to reflux for 10-15 minutes with the reflux condenser at 0°C and open to the atmosphere through a drying tube. After steady state was achieved, samples of the liquid and vapor were taken through sample ports. The temperature of the liquid in the reactor was measure and the vapor temperature was measured at a point between the reactor and the condenser. Duplicate samples were injected into the GC and the relative peak areas were recorded. These relative peak areas converted to mole fractions using the calibration curve.

In the second step, perfluorohexane was charged to a reactor. Subsequently, 2-methyl pentane was added in increments indicated by the data points in the graph. The contents of the reactor were heated after each addition in the same manner as described above for the first step. Samples were taken and analyzed in the same manner as described in the first step. The data collected was then plotted to generate the graph shown in Figure 8.

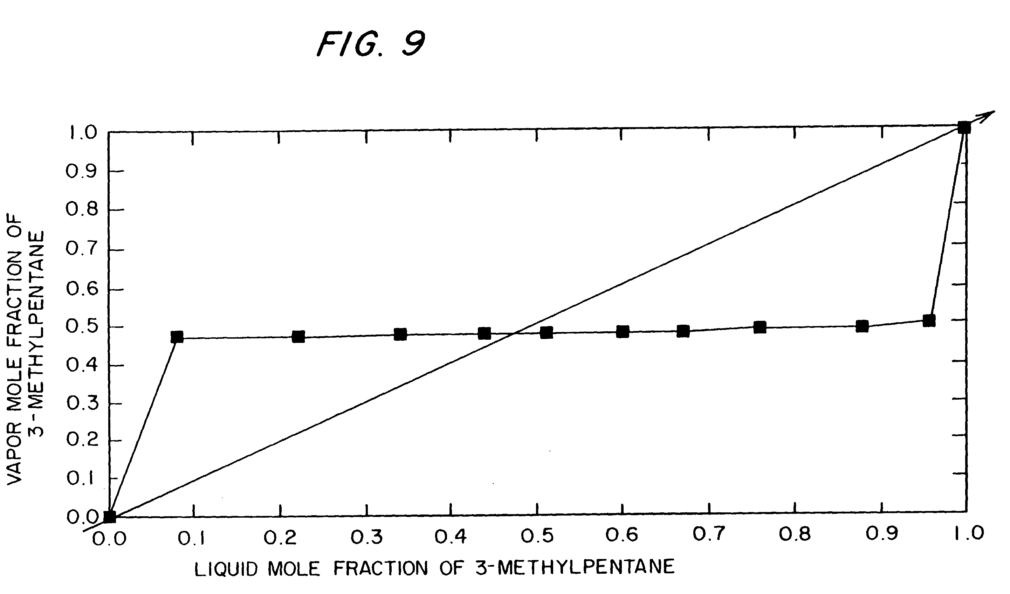

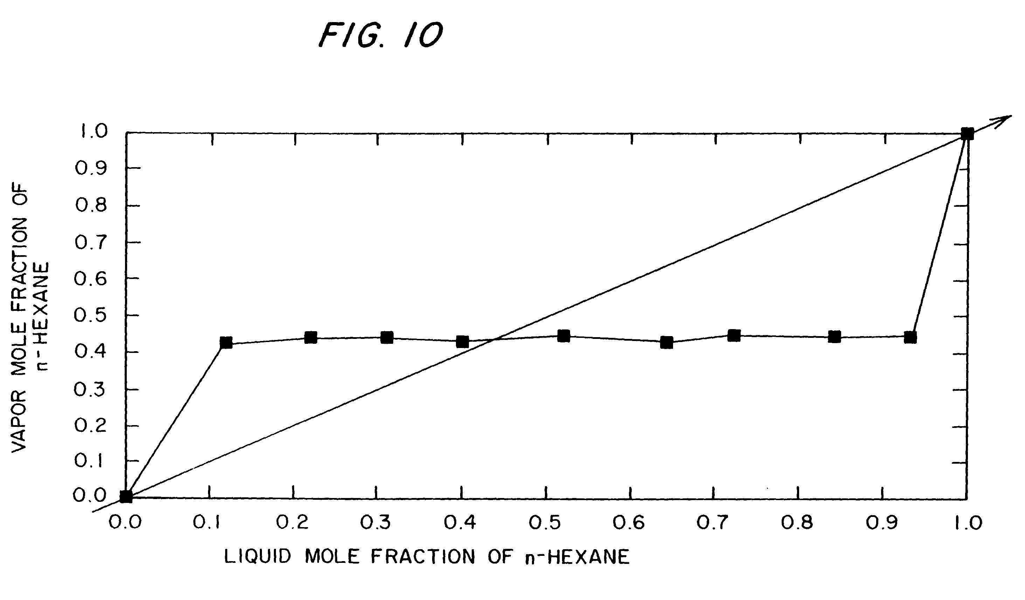

The graphs shown in Figures 9 and 10 were generated in the same manner as Figure 8 using blends of perfluorohexane and 3-methyl pentane (Figure 9) or perfluorohexane and n-hexane (Figure 10).

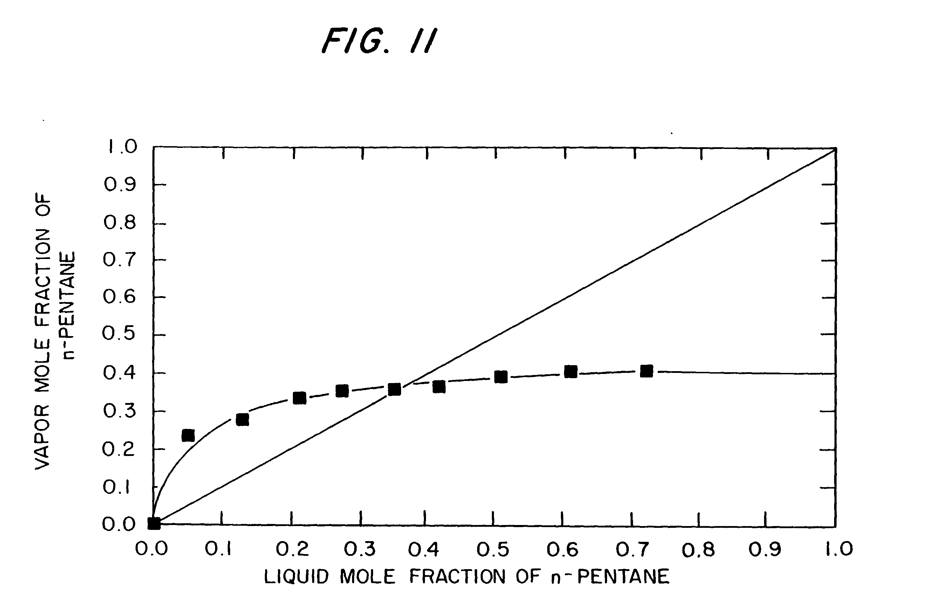

Figure 11 shows a graph on which the mole fraction of n-pentane in the vapor phase is plotted against the mole fraction of n-pentane in the liquid phase of varying mixtures of n-pentane and 1,1,1,4,4,4-hexafluorobutane refluxing at steady state and at one atmosphere. These mole fractions were obtained by gas chromatography and were adjusted to be quantitative by using a calibration curve as is described more fully below. The point at which the mole fraction curve crosses the line with a slope of 1 and intercept 0 is, by definition, the true binary azeotropic composition.

The calibration curve used to calibrate the gas chromatographic results was generated as follows. A series of blends of n-pentane with 1,1,1,4,4,4-hexafluorobutane was prepared with from 0 to 100 mole percent of n-pentane in 10% increments. The mole percent of 1,1,1,-4,4,4-hexafluorobutane in each blend was the difference between 100 mole percent and the mole percent of n-pentane. First, each blend was injected into a GC to establish a correlation between relative peak areas versus actual molar concentrations. This was done by making duplicate samples of each blend and measuring each sample twice. This data was used to establish the calibration curve and a 95% confidence interval which was used to establish the range of error for the azeotropic compositions.

The relative molar amounts of 1,1,1,4,4,4-hexafluorobutane and n-pentane necessary to form an azeotropic composition were then determined by a two step process. In the first step of this process, n-pentane alone was charged to the reactor. Subsequently, 1,1,1,4,4,4-hexafluorobutane was added to the reactor in increments indicated by the data points in the graph. After each addition of 1,1,1,4,4,4-hexafluorobutane, the contents of the reactor were allowed to reflux for 10-15 minutes with the reflux condenser at 0°C and open to the atmosphere through a drying tube. After steady state was achieved, samples of the liquid and vapor were taken through sample ports. The temperature of the liquid in the reactor was measured and the vapor temperature was measured at a point between the reactor and the condenser. Duplicate samples were injected into the GC and the relative peak areas were recorded. These relative peak areas converted to mole fractions using the calibration curve.

In the second step, 1,1,1,4,4,4-hexafluorobutane was charged to a reactor. Subsequently, n-pentane was added in increments indicated by the data points in the graph. The contents of the reactor were then heated and samples were taken and analyzed in the same manner as was done in the first step. The data generated in each of the first and second steps was plotted with the resultant graph being shown in Figure 11.

An azeotrope is defined as a mixture of liquids where, at the boiling point, the concentration of the components is the same in the liquid and vapor phases. The point at which the mole fraction plot crosses the line having a slope of 1 and an intercept of 0 is the expected azeotropic composition.

The azeotropic compositions of the present invention are particularly useful as halogen-free, particularly, chlorine-free, blowing agents for the production of rigid foams. The amount of azeotropic composition used depends on the desired foam density and the presence of additional blowing agents (the water) and has to be adjusted according to methods known to those skilled in the art. Typically, these compositions are generally included in the foam-forming mixture in an amount of from about 1% to about 20% preferably from about 2% to about 12%. The ability of these azeotropic compositions to act as a blowing agent is particularly surprising in view of the fact that pure perfluorohexane does not produce a useful foam under the conditions used in the Examples which follow.

Foams made with the 1,1,1,4,4,4-hexafluorobutane-containing azeotropic compositions of the present invention are comparable to or, in some cases, substantially lower in density than foams produced with R-356 alone but still have relatively low K-factors.

Foams made with the azeotropic compositions of the present invention contain no CFCs, HCFCs, FCs or HFCs but still have relatively low K-factors.

Foams may be produced with the azeotropic compositions of the present invention by reacting a) an isocyanate-reactive material with b) an organic polyisocyanate in the presence of one of the azeotropic compositions of the present invention, optionally in the presence of a catalyst, foam stabilizer, or other additives, auxiliary agents and processing aids.

Any of the known isocyanate-reactive materials, organic polyisocyanates (as used herein, "polyisocyanates" includes diisocyanates), catalysts and foam stabilizers may be used to produce foams with the azeotropic compositions of the present invention.

The known isocyanates which are useful in the practice of the present invention include polyisocyanates, modified polyisocyanates and isocyanate-terminated prepolymers. Suitable polyisocyanates which may be used to produce foams in accordance with the present invention include aromatic, aliphatic, cycloaliphatic polyisocyanates and combinations thereof. Specific examples of such isocyanates include: diisocyanates such as m-phenylene diisocyanate, p-phenylene diisocyanate, 2,4-toluene diisocyanate, 2,6-toluene diisocyanate, 1,6-hexamethylene diisocyanate, 1,4-hexamethylene diisocyanate, 1,4-cyclohexane diisocyanate, hexahydrotoluene diisocyanate and its isomers, 1,5-naphthylene diisocyanate, 1-methylphenyl-2,4-phenyl diisocyanate, 4,4'-diphenylmethane diisocyanate, 2,4'-diphenylmethane diisocyanate, 4,4'-biphenylene diisocyanate, 3,3'-dimethoxy-4,4'-biphenylene diisocyanate, and 3,3'-dimethyl-diphenylpropane-4,4'-diisocyanate; triisocyanates such as 2,4,6-toluene triisocyanate; and polyisocyanates such as 4,4'-dimethyl-diphenylmethane-2,2',5,5'-tetraisocyanate and the polymethylene polyphenylisocyanates.

Isocyanate-terminated prepolymers having an NCO content of at least about 8%, preferably from about 9 to about 30%, are particularly useful for producing polyurethane foams in accordance with the present invention. Prepolymers of diphenylmethane diisocyanate having NCO contents of from about 8 to about 17%, preferably about 10%, by weight, are particularly preferred. These preferred prepolymers may be made, for example, by pre-reacting diphenylmethane diisocyanate (MDI) or an isomer mixture of MDI with an isocyanate-reactive compound such as a polyol or polyamine having a functionality of from about 1.9 to about 3.1, preferably about 2 in an amount such that the unreacted isocyanate group content is within the above-specified range.

Among the most preferred polyisocyanates for the production of rigid polyurethanes are methylene-bridged polyphenyl polyisocyanates and prepolymers of methylene-bridged polyphenyl polyisocyanates having an average functionality of from about 1.8 to about 3.5 (preferably from about 2.0 to about 3.1) isocyanate moieties per molecule and an NCO content of from about 25 to about 35% by weight, due to their ability to crosslink the polyurethane.

Any of the catalysts known to be useful in the production of rigid polyurethane foams may be employed in the practice of the present invention. Tertiary amine catalysts are particularly preferred. Specific examples of suitable catalysts include: pentamethyl-diethylene triamine, N,N-dimethyl-cyclohexyl amine, N,N',N"-dimethylamino-propylhexahydrotriazine, tetramethylene diamine, tetramethyl-butylene diamine, and dimethylethanolamine. Pentamethyl-diethylene triamine, N,N',N"-dimethyl-amino-propyl-hexahydrotriazine, and N,N-dimethyl-cyclohexyl amine are particularly preferred.

Isocyanate-terminated prepolymers having an NCO content of at least about 25%, preferably from about 27 to about 33%, may also be used to produce foams in accordance with the present invention. Prepolymers of diphenylmethane diisocyanate having NCO contents of from about 25 to about 35%, preferably about 29%, by weight, are particularly preferred. These preferred prepolymers are made by pre-reacting diphenylmethane diisocyanate (MDI) or an isomer mixture of MDI with an isocyanate-reactive compound such as a polyol or polyamine having a functionality of from about 1.0 to about 4.0, preferably about 2 in an amount such that the unreacted isocyanate group content is within the above-specified range.

Undistilled or crude polyisocyanates may also be used in the practice of the present invention. The crude toluene diisocyanate obtained by phosgenating a mixture of toluene diamines and the crude diphenylmethane diisocyanate obtained by phosgenating crude diphenylmethane diamine (polymeric MDI) are examples of suitable crude polyisocyanates. Suitable undistilled or crude polyisocyanates are disclosed in U.S. Patent 3,215,652.

Suitable modified polyisocyanates may be obtained by chemical reaction of diisocyanates and/or polyisocyanates. Modified isocyanates useful in the practice of the present invention include isocyanates containing ester groups, urea groups, biuret groups, allophanate groups, carbodiimide groups, isocyanurate groups, uretdione groups and/or urethane groups. Preferred examples of modified isocyanates include prepolymers containing isocyanate groups and having an isocyanate group content of from about 25 to about 35% by weight, preferably from about 27 to about 33% by weight, most preferably 28 to 32% by weight, particularly those based on polyether polyols or polyester polyols and diphenylmethane diisocyanate. Processes for producing these modified polyisocyanates are known in the art.

The polyisocyanate, modified polyisocyanate or isocyanate-terminated prepolymer may be reacted with any of the polyols or polyamines which are known to be useful in the production of polyurethane/polyurea foams. Suitable polyols include polyether polyols, polyester polyols, polyhydroxy polyacetals, polyhydroxy polycarbonates, polyhydroxy polyacrylates, polyester ethers, polyhydroxy polythioethers, polyhydroxy polyester amides, polybutadienes and polylactones having a molecular weight of from about 400 to about 10,000 and a functionality of at least about two, preferably from about 2 to about 4. Polyester polyols, polyether polyols and mixtures of such polyols are preferred.

Suitable polyester polyols include the reaction products of polyhydric alcohols (preferably dihydric alcohols to which trihydric alcohols may be added) and polybasic (preferably dibasic) carboxylic acids. In addition to these polycarboxylic acids, corresponding carboxylic acid anhydrides or polycarboxylic acid esters of lower alcohols or mixtures thereof may also be used to prepare the polyester polyols useful in the practice of the present invention. The polycarboxylic acids may be aliphatic, cycloaliphatic, aromatic and/or heterocyclic and they may be substituted, e.g. by halogen atoms, and/or unsaturated. Examples of suitable polycarboxylic acids include: succinic acid; adipic acid; suberic acid; azelaic acid; sebacic acid; phthalic acid; isophthalic acid; trimellitic acid; phthalic acid anhydride; tetrahydrophthalic acid anhydride; hexahydrophthalic acid anhydride; tetrachlorophthalic acid anhydride, endomethylene tetrahydrophthalic acid anhydride; glutaric acid anhydride: maleic acid: maleic acid anhydride; fumaric acid; dimeric and trimeric fatty acids such as oleic acid, which may be mixed with monomeric fatty acids; dimethyl terephthalates and bis-glycol terephthalates. Suitable polyhydric alcohols include: ethylene glycol; 1,2- and 1,3-propylene glycol; 1,3- and 1,4-butylene glycol; 1,6-hexanediol; 1,8-octanediol; neopentyl glycol; cyclohexanedimethanol; (1,4-bis(hydroxymethyl)-cyclohexane); 2-methyl-1,3-propanediol; 2,2,4-trimethyl-1,3-pentanediol; triethylene glycol; tetraethylene glycol; polyethylene glycol; dipropylene glycol; polypropylene glycol; dibutylene glycol and polybutylene glycol, glycerine and trimethylolpropane. The polyesters may also contain a portion of carboxyl end groups. Polyesters of lactones, e.g. ε-caprolactone or hydroxyl carboxylic acids such as ω-hydroxycaproic acid, may also be used.

Suitable polycarbonates containing hydroxyl groups include those obtained by reacting diols with phosgene, a diarylcarbonate (e.g., diphenyl carbonate) or cyclic carbonates (e.g., ethylene or propylene carbonate). Examples of suitable diols include: 1,3-propanediol; 1,4-butanediol; 1,6-hexanediol; diethylene glycol; triethylene glycol; and tetraethylene glycol. Polyester carbonates obtained by reacting polyesters or polylactones (such as those described above) with phosgene, diaryl carbonates or cyclic carbonates may also be used in the practice of the present invention.

Polyether polyols which are suitable for producing foams in accordance with the present invention include those obtained in known manner by reacting one or more starting compounds which contain reactive hydrogen atoms with alkylene oxides such as ethylene oxide, propylene oxide, butylene oxide, styrene oxide, tetrahydrofuran, epichlorohydrin or mixtures of these alkylene oxides. Polyethers which do not contain more than about 10% by weight of ethylene oxide units are preferred. Polyethers obtained without the addition of ethylene oxide are most preferred. Suitable starting compounds containing reactive hydrogen atoms include polyhydric alcohols (described above as being suitable for preparing polyester polyols); water; methanol; ethanol; 1,2,6-hexane triol; 1,2,4-butane triol; trimethylol ethane; pentaerythritol; mannitol; sorbitol; methyl glycoside; sucrose; phenol; isononyl phenol; resorcinol; hydroquinone; and 1,1,1- or 1,1,2-tris-(hydroxylphenyl)ethane.

Amine-initiated polyether polyols having functionalities of from about 3 to about 4 and molecular weights of at least about 149, preferably from about 149 to about 1500, most preferably from about 300 to about 800 are also useful. These amine-based polyols may be prepared by reacting an amine, polyamine or aminoalcohol and optionally other initiators (with or without water) with propylene oxide and optionally, ethylene oxide, in the presence of an alkaline catalyst. The product is then treated with an acid, preferably a hydroxy-carboxylic acid to neutralize the alkaline catalyst. U.S. Patent 2,697,118 discloses a suitable process for the production of such amine-initiated polyols.

Examples of suitable amine initiators include: ammonia, ethylene diamine, diethylene triamine, hexamethylene diamine, amines such as toluene diamine, and aminoalcohols. Aminoalcohols, particularly, monoethanolamine, diethanolamine, and triethanolamine are preferred initiators.

It is preferred that the amine initiator be reacted with propylene oxide, although it may also be reacted with ethylene oxide. If used, the ethylene oxide may be used in an amount of up to 100% by weight of the total alkylene oxide used. The propylene oxide is generally used in an amount of from about 40 to about 100% by weight of the total alkylene oxide employed, preferably from about 60 to about 100% by weight. The total amount of alkylene oxide used is selected so that the product polyol will have an average molecular weight of at least about 149, preferably from about 149 to about 1500.

The amine-based polyether polyol is included in the foam-forming mixture in an amount of from about 20 to about 70% by weight, based on the total foam forming mixture, preferably from about 40 to about 50% by weight.

Other polyether polyols (i.e., polyether polyols which are not based on an amine) known to be useful in the production of rigid polyurethane foams as well as polyester polyols may also be used in the practice of the present invention. Combinations of an amine-initiated polyol and polyols which are not based upon amines are particularly preferred. When such mixtures are used, the amine-initiated polyol is generally included in an amount of at least 20% by weight, preferably from about 50 to about 80% by weight.

When the amine-initiated polyol is based upon an aminoalcohol, polyester polyols having functionalities of from about 2 to about 3 (preferably from about 2 to about 2.5) and molecular weights of from about 180 to about 900, preferably from about 300 to about 600 are preferably included in the polyol mixture in an amount of from about 5 to about 50%, most preferably from about 15 to about 35% by weight of the total amount of polyol.

Polyethers modified by vinyl polymers are also suitable for producing foams in accordance with the present invention. Such modified polyethers may be obtained, for example, by polymerizing styrene and acrylonitrile in the presence of a polyether (U.S. Patent Nos. 3,383,351; 3,304,273; 3,523,095; 3,110,695 and German Patent No. 1,152,536).

The polythioethers useful in the production of foams in accordance with the present invention include the condensation products obtained from thiodiglycol on its own and/or with other glycols, dicarboxylic acids, formaldehyde, aminocarboxylic acids or amino alcohols. These condensation products may be polythio-mixed ethers, polythioether esters or polythioether ester amides, depending on the co-components.

Amine-terminated polyethers may also be used to produce foams in accordance with the present invention. Suitable amine-terminated polyethers may be prepared by reacting a primary amine with a polyether containing terminal leaving group such as halides, or mesylates as disclosed in commonly assigned U.S. Patent Application Serial Number 07/957,929, filed on October 7, 1992, or as disclosed in U.S. Patents 3,666,726, 3,691,112 and 5,066,824.

Suitable polyacetals include those prepared from aldehydes (e.g., formaldehyde) and glycols such as diethylene glycol, triethylene glycol, ethoxylated 4,4'-dihydroxydiphenyldimethylmethane, and 1,6-hexanediol. Polyacetals prepared by the polymerization of cyclic acetals may also be used in the practice of the present invention.

Polyhydroxy polyester amides and polyamines useful in the production of foams with the blowing agents of the present invention include the predominantly linear condensates obtained from polybasic saturated and unsaturated carboxylic acids or their anhydrides and polyvalent saturated or unsaturated aminoalcohols, diamines, polyamines and mixtures thereof.

Suitable monomers for producing hydroxy-functional polyacrylates include acrylic acid, methacrylic acid, crotonic acid, maleic anhydride, 2-hydroxyethyl acrylate, 2-hydroxyethyl methacrylate, 2-hydroxypropyl acrylate, 2-hydroxypropyl methacrylate, 3-hydroxypropyl acrylate, 3-hydroxypropyl methacrylate, glycidyl acrylate, glycidyl methacrylate, 2-isocyanatoethyl acrylate and 2-isocyanatoethyl methacrylate.

Low molecular weight, isocyanate-reactive compounds useful in producing foams in accordance with the present invention have from 2 to 8 hydroxyl groups, preferably 2 to 4 and most preferably two hydroxyl groups, and have an average molecular weight of from about 34 to about 399, preferably from about 60 to about 200, most preferably from about 100 to about 150 and may be used in combination with or instead of the high molecular weight material containing two or more hydroxyl groups. Useful low molecular weight isocyanate-reactive materials include the polyhydric alcohols which have previously been described in the process for the preparation of the polyester polyols and polyether polyols. Dihydric alcohols are preferred. The weight ratio of the low molecular weight to the high molecular weight material containing two or more hydroxyl groups is not critical to the invention. Ratios of from about 1:100 to about 1:10, preferably from about 2:100 to about 5:100, have been found to be useful.

In addition to the above-mentioned isocyanate-reactive compounds, monofunctional and even small amounts of trifunctional and higher functional compounds generally known in polyurethane chemistry may be used to produce foams in accordance with the present invention. For example, trimethylolpropane may be used in special cases in which slight branching is desired.

Catalysts and solvents may be used to aid the reaction. Examples of catalysts useful for promoting urethane reactions include di-n-butyl tin dichloride, di-n-butyl tin diacetate, di-n-butyl tin dilaurate, triethylenediamine, or bismuth nitrate. Tertiary amine catalysts are particularly preferred. Specific examples of suitable catalysts include: pentamethyl-diethylene triamine, N,N-dimethyl-cyclohexyl amine, N,N',N"-dimethyl-amino-propyl-hexahydro triazine, tetramethylene diamine, tetramethyl-butylene diamine, and dimethylethanolamine. Pentamethyl-diethylene triamine, N,N',N"-dimethylamino-propyl-hexahydro triazine, and N,N-dimethyl-cyclohexyl amine are particularly preferred.

Materials which may optionally be included in the foam-forming mixtures of the present invention include: chain extenders, crosslinking agents, surfactants, pigments, colorants, fillers, antioxidants, flame retardants, light stabilizers, heat stabilizers, UV stabilizers, and cell openers. Carbon black is a preferred additive.

Any of the known methods for producing polyurethane foams may be used in the practice of the present invention. Suitable methods include reaction of the various reactants using the known one-shot process, prepolymer process or semi-prepolymer process.

Having thus described our invention, the following Examples are given as being illustrative thereof. All parts and percentages given in these Examples are parts by weight or percentages by weight, unless otherwise indicated.

### EXAMPLES

The following materials were used in the Examples:
- POLYOL A:: A 630 OH number polyol prepared by reacting 1 mole of ethylene diamine with 5 moles of propylene oxide.
- POLYOL B:: A 250 OH number polyol prepared by reacting 1 mole of glycerin with approximately 3.3 moles of propylene oxide.
- 2-MeB:: 2-methyl butane.
- n-P:: n-pentane.
- Tegostab B-8426:: A polysiloxane polyether copolymer which is commercially available from Goldschmidt Chemical Corporation.
- DMCHA:: dimethylcyclohexylamine.
- ISO:: The polymethylene polyphenyl polyisocyanate prepolymer having an NCO content of approximately 27% which is commercially available from Miles Inc. under the name Mondur E-577.
- PFH:: perfluorohexane.
- R-356:: 1,1,1,4,4,4-hexafluorobutane.
- 1,3-Diox:: 1,3-dioxolane.
- 2-MeB:: 2-methyl butane.
- n-P:: n-pentane.
- CP:: cyclopentane.
- 2-MeP:: 2-methylpentane.
- 3-MeP:: 3-methylpentane.
- n-Hex:: n-hexane.

### EXAMPLE 1

7.59 parts PFH and 7.89 parts 2-MeB were first mixed. This azeotropic mixture was then blended with the other components listed in TABLE 1 under B-SIDE. (The materials and the amount of each of those materials included in the B-SIDE are given in TABLE 1.) The amount of ISO indicated in TABLE 1 was then mixed with the B-SIDE in a mixing vessel using an air driven stirrer. After 5 seconds of mixing, the reaction mixture was poured into an aluminum mold heated to 40°C, which mold measured 14"x14"x 3". The reactivity time, density and K-factor of the foam produced were determined. The results of these determinations are reported in TABLE 1.

### EXAMPLE 2

The procedure of Example 1 was repeated using the same materials with the exception that a different azeotrope was used. The azeotrope used in this Example was made with 11.89 parts PFH and 7.29 parts n-P. The amounts of the specific materials used and the characteristics of the product foam are reported in TABLE 1.

### EXAMPLE 3 (COMPARATIVE)

The procedure of Example 1 was repeated using the same materials with the exception that 61.07 parts by weight of PFH alone (rather than an azeotrope) was used as the blowing agent. The specific materials, and the amount of each material are all reported in TABLE 1.

The resulting foam showed an irregular cell structure and large voids and was not useful. No foam properties could be reported.

**TABLE 1**

| Example | 1 | 2 | 3 |
|---|---|---|---|
| **B-SIDE** | | | |
| POLYOL A (pbw) | 35.89 | 35.89 | 35.89 |
| POLYOL B (pbw) | 35.89 | 35.89 | 35.89 |
| Tegostab B-8426 (pbw) | 1.59 | 1.59 | 1.59 |
| Water (pbw) | 1.59 | 1.59 | 1.59 |
| DMCHA (pbw) | 2.62 | 2.62 | 2.62 |
| PFH (pbw) | 7.59 | 11.89 | 61.07 |
| 2-MeB (pbw) | 7.89 | ----- | ----- |
| n-P (pbw) | ----- | 7.29 | ----- |

| **A-SIDE** | | | |
|---|---|---|---|
| ISO (pbw) | 117.28 | 117.28 | 117.28 |

| **RESULTS** | | | |
|---|---|---|---|
| Mix Time (sec) | 5 | 5 | 5 |
| Cream Time (sec) | <10 | <10 | <10 |
| Gel Time (sec) | 38 | 37 | ----- |
| Density (lb/ft³) | 1.87 | 1.83 | ----- |
| K-factor (BTU-in./°F hr.ft²) | 0.151 | 0.199 | ----- |

### EXAMPLE 4

16.53 parts of R-356 and 6.43 parts of 2-MB were first mixed. This mixture was then blended with the other components listed in TABLE 2 under B-SIDE. (The materials and the amount of each of those materials included in the B-SIDE are given in TABLE 2.) 116.5 parts of ISO were then mixed with the B-SIDE in a mixing vessel using an air driven stirrer. After 5 seconds of mixing, the reaction mixture was poured into a polyethylene-lined cardboard box which measured 10"x10"x 2.5". The reactivity time, density and K-factor of the foam produced were determined. The results of these determinations are reported in TABLE 2.

### EXAMPLE 5 (COMPARATIVE):

The procedure of Example 4 was repeated using the same materials with the exception that only R-356 was used as the blowing agent. The specific materials, the amount of each material and the characteristics of the product foam are all reported in TABLE 2.

**TABLE 2**

| Example | 4 | 5 |
|---|---|---|
| **B-SIDE** | | |
| POLYOL A (pbw) | 35.64 | 35.64 |
| POLYOL B (pbw) | 35.64 | 35.64 |
| Tegostab B-8426 (pbw) | 1.58 | 1.58 |
| Water, pbw | 1.58 | 1.58 |
| DMCHA, pbw | 2.60 | 2.60 |
| R-356, pbw | 16.53 | 22.96 |
| 2-MB, pbw | 6.43 | ----- |

| **A-SIDE** | | |
|---|---|---|
| ISO, pbw | 116.5 | 116.5 |

| **RESULTS** | | |
|---|---|---|
| Mix Time (sec) | 5 | 5 |
| Cream Time (sec) | 6 | 6 |
| Gel Time (sec) | 51 | 46 |
| Density (lb/ft³) | 1.48 | 1.80 |
| K-factor (BTU-in./°F hr.ft²) | 0.141 | 0.130 |

### EXAMPLE 6

12.93 parts of CP and 2.10 parts of 1,3-Diox were first mixed. This mixture was then blended with the other components listed in TABLE 3 under B-SIDE. (The materials and the amount of each of those materials included in the B-SIDE are given in TABLE 3.) The amount of ISO indicated in TABLE 3 was then mixed with the B-SIDE in a mixing vessel using an air driven stirrer. After 5 seconds of mixing, the reaction mixture was poured into a polyethylene-lined cardboard box which measured 14"x14"x 3". The reactivity time, density and K-factor of the foam produced were determined. The results of these determinations are reported in TABLE 3.

### EXAMPLE 7 (not according to the invention)

The procedure of Example 6 was repeated using the same materials with the exception that a different azeotrope was used. The azeotrope used in this Example was made with 11.88 parts by weight 2-MeP and 5.09 parts by weight of 1,3-Diox. The amounts of the specific materials used and the characteristics of the product foam are reported in TABLE 3.

### EXAMPLE 8

The procedure of Example 6 was repeated using the same materials with the exception that a different azeotrope was used. The azeotrope used in this Example was made with 11.51 parts by weight 3-MeP and 5.41 parts by weight 1,3-Diox. The amounts of the specific materials used and the characteristics of the product foam are reported in TABLE 3.

### EXAMPLE 9

The procedure of Example 6 was repeated using the same materials with the exception that a different azeotrope was used. The azeotrope used in this Example was made with 10.37 parts by weight n-Hex and 6.92 parts by weight 1,3-Diox. The amounts of the specific materials used and the characteristics of the product foam are reported in TABLE 3.

### EXAMPLE 10 (COMPARATIVE)

The procedure of Example 6 was repeated using the same materials with the exception that 14.40 parts by weight of CP alone (rather than an azeotrope) was used as the blowing agent. The specific materials, the amount of each material and the characteristics of the product foam are all reported in TABLE 3.

**TABLE 3**

| Example | 6 | 7 | 8 | 9 | 10 |
|---|---|---|---|---|---|
| **B-SIDE** | | | | | |
| POLYOL A, pbw | 61.69 | 61.34 | 61.35 | 61.28 | 61.81 |
| POLYOL B, pbw | 61.69 | 61.34 | 61.35 | 61.28 | 61.81 |
| Tegostab B-8426, pbw | 2.73 | 2.72 | 2.72 | 2.71 | 2.74 |
| Water, pbw | 2.73 | 2.72 | 2.72 | 2.71 | 2.74 |
| DMCHA, pbw | 4.50 | 4.48 | 4.48 | 4.47 | 4.51 |
| CP, pbw | 12.93 | ---- | ---- | ---- | 14.40 |
| 1,3-Diox, pbw | 2.10 | 5.09 | 5.41 | 6.92 | ---- |
| 1-MeP, pbw | ---- | 11.88 | ---- | ---- | ---- |
| 3-MeP, pbw | ---- | ---- | 11.51 | ---- | ---- |
| n-Hex, pbw | ---- | ---- | ---- | 10.37 | ---- |

| **A-SIDE** | | | | | |
|---|---|---|---|---|---|
| ISO, pbw | 201.61 | 200.44 | 200.47 | 200.25 | 202 |

| PROPS. | | | | | |
|---|---|---|---|---|---|
| Mix Time (sec) | 5 | 5 | 5 | 5 | 5 |
| Cream Time (sec) | <10 | <10 | <10 | <10 | <10 |
| Gel Time (sec) | 34 | 35 | 35 | 32 | 36 |
| Density (lb/ft³) | 1.94 | 2.03 | 2.03 | 2.09 | 1.92 |
| K-Factor (BTU-in. /°F hr.ft²) | 0.131 | 0.144 | 0.142 | 0.144 | 0.132 |

### EXAMPLE 11 (not according to the invention)

39.62 parts PFH and 9.90 parts 2-MeP were first mixed. This mixture was then blended with the other components listed in TABLE 1 under B-SIDE. (The materials and the amount of each of those materials included in the B-SIDE are given in TABLE 4.) The amount of ISO indicated in TABLE 1 was then mixed with the B-SIDE in a mixing vessel using an air driven stirrer. After 5 seconds of mixing, the reaction mixture was poured into an aluminum mold heated to 40°C which mold measured 14"x14"x 3". The reactivity time, density and K-factor of the foam produced were determined. The results of these determinations are reported in TABLE 4.

### EXAMPLE 12

The procedure of Example 11 was repeated using the same materials with the exception that a different azeotrope was used. The azeotrope used in this Example was made with 41.69 parts PFH and 9.15 parts 3-MeP. The amounts of the specific materials used and the characteristics of the product foam are reported in TABLE 4.

### EXAMPLE 13

The procedure of Example 11 was repeated using the same materials with the exception that a different azeotrope was used. The azeotrope used in this Example was made with 43.81 parts PFH and 8.97 parts n-Hex. The amounts of the specific materials used and the characteristics of the product foam are reported in TABLE 4.

### EXAMPLE 14 (COMPARATIVE)

The procedure of Example 11 was repeated using the same materials with the exception that 61.07 parts by weight of PFH alone (rather than an azeotrope) was used as the blowing agent. The specific materials, the amount of each material and the characteristics of the product foam are all reported in TABLE 4. The resulting foam showed an irregular cell structure and large voids and was not useful. No foam properties could be reported.

**TABLE 4**

| Example | 11 | 12 | 13 | 14 |
|---|---|---|---|---|
| **B-SIDE** | | | | |
| POLYOL A (pbw) | 55.34 | 55.10 | 54.74 | 35.89 |
| POLYOL B (pbw) | 55.34 | 55.10 | 54.74 | 35.89 |
| Tegostab B-8426 (pbw) | 2.45 | 2.44 | 2.43 | 1.59 |
| Water (pbw) | 2.45 | 2.44 | 2.43 | 1.59 |
| DMCHA (pbw) | 4.04 | 4.02 | 4.00 | 2.62 |
| PFH (pbw) | 39.62 | 41.69 | 43.81 | 61.07 |
| 2-MeP (pbw) | 9.90 | ---- | ---- | ---- |
| 3-MeP (pbw) | ---- | 9.15 | ---- | ---- |
| n-Hex (pbw) | ---- | ---- | 8.97 | ---- |

| **A-SIDE** | | | | |
|---|---|---|---|---|
| ISO (pbw) | 180.85 | 180.06 | 178.89 | 117.28 |
| **RESULTS** | | | | |
| Mix Time (sec) | 5 | 5 | 5 | 5 |
| Cream Time (sec) | <10 | <10 | <10 | <10 |
| Gel Time (sec) | 35 | 37 | 36 | ---- |
| Density (lb/ft³) | 1.79 | 1.77 | 1.77 | ---- |
| K-factor (BTU-in./°F hr.ft.²) | 0.198 | 0.206 | 0.208 | ---- |

### EXAMPLE 15

19.91 parts of R-356 and 4.98 parts of n-P were first mixed. This mixture was then blended with the other components listed in TABLE 5 under B-SIDE. (The materials and the amount of each of those materials included in the B-SIDE are given in TABLE 5.) ISO was then mixed with the B-SIDE (in the amount indicated in TABLE 5) in a mixing vessel using an air driven stirrer. After 5 seconds of mixing, the reaction mixture was poured into a polyethylene-lined cardboard box which measured 10"x10"x 2.5". The reactivity time, density and K-factor of the foam produced were determined. The results of these determinations are reported in TABLE 5.

### EXAMPLE 16 (COMPARATIVE)

The procedure of Example 15 was repeated using the same materials with the exception that only R-356 was used as the blowing agent. The specific materials, the amount of each material and the characteristics of the product foam are all reported in TABLE 5.

**TABLE 5**

| EXAMPLE | 15 | 16 |
|---|---|---|
| **B-SIDE** | | |
| POLYOL A, pbw | 50.67 | 49.56 |
| POLYOL B, pbw | 50.67 | 49.56 |
| Tegostab B-8426, pbw | 2.24 | 2.20 |
| Water, pbw | 2.24 | 2.20 |
| DMCHA, pbw | 3.70 | 3.26 |
| R-356, pbw | 19.91 | 30.75 |
| n-P, pbw | 4.98 | ----- |

| **A-SIDE** | | |
|---|---|---|
| ISO, pbw | 165.58 | 162.06 |

| **RESULTS** | | |
|---|---|---|
| Mix Time, sec. | 5 | 5 |
| Cream Time, sec. | <10 | <10 |
| Gel Time, sec. | 46 | 46 |
| density, lbs/ft³ | 1.86 | 1.82 |
| K-Factor (BTU-in./°F hr.ft²) | 0.133 | 0.127 |

Although the invention has been described in detail in the foregoing for the purpose of illustration, it is to be understood that such detail is solely for that purpose and that variations can be made therein by those skilled in the art without departing from the spirit and scope of the invention except as it may be limited by the claims.

## Claims

1. An azeotropic composition consisting essentially of
a) (1) from 26 to 72% by weight perfluorohexane
and
b) one compound selected from the group consisting of
(1) from 36 to 74% by weight 2-methyl butane or
(2) from 28 to 53% by weight n-pentane
in which the sum of the weight percent a) plus weight percent b) is 100 percent.

2. An azeotropic composition consisting essentially of
a)(3) from 64 to 80% by weight of 1,1,1,4,4,4-hexafluorobutane
and
b)(1) from 20 to 36% by weight of 2-methyl butane.

3. An azeotropic composition consisting essentially of
a)(2) from 4 to 50% by weight 1,3-dioxolane
and
b) one compound selected from the group consisting of
(3) from 75 to 96% by weight cyclopentane or
(4) from 59 to 77% by weight 3-methyl pentane or
(5) from 50 to 69% by weight n-hexane
in which the sum of the weight percent a) plus weight percent b) is 100 percent.

4. An azeotropic composition consisting essentially of
a)(1) from 73 to 88% by weight perfluorohexane
and
b) one compound selected from the group consisting of
(4) from 13 to 25% by weight 3-methyl pentane or
(5) from 12 to 23% by weight n-hexane
in which the sum of the weight percent a) plus weight percent b) is 100 percent.

5. An azeotropic composition consisting essentially of
a)(3) from 73 to 87% by weight of 1,1,1,4,4,4-hexafluorobutane and
b)(2) from 13 to 27% by weight of n-pentane.

6. A process for the production of a polyurethane foam comprising reacting a polyisocyanate with an isocyanate-reactive material in the presence of the azeotropic composition of one of claims 1 to 5.

7. A polyurethane foam which has been produced by reacting a polyisocyanate with an isocyanate reactive material in the presence of the azeotropic composition of one of claims 1 to 5.

## Patentansprüche

1. Azeotrope Zusammensetzung, im wesentlichen bestehend aus:
a) (1) 26 bis 72 Gew.-% Perfluorhexan und
b) einer Verbindung, die aus der Gruppe ausgewählt ist, die aus
(1) 36 bis 74 Gew.-% 2-Methylbutan oder
(2) 28 bis 53 Gew.-% n-Pentan besteht,
worin die Summe der Gew.-% von a) plus der Gew.- % von b) 100 % ist.

2. Azeotrope Zusammensetzung, die im wesentlichen aus
a)(3) 64 bis 80 Gew.-% 1,1,1,4,4,4-Hexafluorbutanund
b)(1) 20 bis 36 Gew.-%2-Methylbutan
besteht.

3. Azeotrope Zusammensetzung, im wesentlichen bestehend aus:
a)(2) 4 bis 50 Gew.-% 1,3-Dioxolan und
b) einer Verbindung, die aus der Gruppe ausgewählt ist, die aus
(3) 75 bis 96 Gew.-% Cyclopentan oder
(4) 59 bis 77 Gew.-% 3-Methylpentan oder
(5) 50 bis 69 Gew.-% n-Hexan besteht,
worin die Summe der Gew.-% von a) plus der Gew.-% von b) 100 % ist.

4. Azeotrope Zusammensetzung, im wesentlichen bestehend aus:
a)(1) 73 bis 88 Gew.-% Perfluorhexan und
b) einer Verbindung, die aus der Gruppe ausgewählt ist, die aus
(4) 13 bis 25 Gew.-% 3-Methylpentan oder
(5) 12 bis 23 Gew.-% n-Hexan besteht,
worin die Summe der Gew.-% von a) plus der Gew.-% von b) 100 % ist.

5. Azeotrope Zusammensetzung, die im wesentlichen aus
a)(3) 73 bis 87 Gew.-% 1,1,1,4,4,4-Hexafluorbutan und
b)(2) 13 bis 27 Gew.-% n-Pentan
besteht.

6. Verfahren zur Herstellung eines Polyurethanschaums, umfassend die Umsetzung eines Polyisocyanats mit einem isocyanatreaktiven Material in Gegenwart der azeotropen Zusammensetzung gemäß einem der Ansprüche 1 bis 5.

7. Polyurethanschaum, der durch Umsetzung eines Polyisocyanats mit einem isocyanatreaktiven Material in Gegenwart der azeotropen Zusammensetzung gemäß einem der Ansprüche 1 bis 5 hergestellt wird.

## Revendications

1. Composition azéotrope constituée essentiellement
a) (1) de 26 à 72% en poids de perfluorohexane
et
b) d'un composé choisi dans le groupe constitué par
(1) de 36 à 74% en poids de 2-méthylbutane ou
(2) de 28 à 53% en poids de n-pentane,
dans laquelle la somme du pourcentage en poids de a) plus le pourcentage en poids de b) est 100 pour-cent.

2. Composition azéotrope constituée essentiellement
a) (3) de 64 à 80% en poids de 1,1,1,4,4,4-hexafluorobutane
et
b) (1) de 20 à 36% en poids de 2-méthylbutane.

3. Composition azéotrope constituée essentiellement de
a) (2) de 4 à 50% en poids de 1,3-dioxolane
et
b) d'un composé choisi dans le groupe constitué par
(3) de 75 à 96% en poids de cyclopentane
ou
(4) de 59 à 77% en poids de 3-méthylpentane ou
(5) de 50 à 69% en poids de n-hexane,
dans laquelle la somme du pourcentage en poids de a) plus le pourcentage en poids de b) est 100 pour-cent.

4. Composition azéotrope constituée essentiellement
a) (1) de 73 à 88% en poids de perfluorohexane
et
b) d'un composé choisi dans le groupe constitué de
(4) de 13 à 25% en poids de 3-méthylpentane ou
(5) de 12 à 23% en poids de n-hexane,
dans laquelle la somme du pourcentage en poids de a) plus le pourcentage en poids de b) est 100 pour-cent.

5. Composition azéotrope constituée essentiellement
a) (3) de 73 à 87% en poids de 1,1,1,4,4,4-hexafluorobutane et
b) (2) de 13 à 27% en poids de n-pentane.

6. Procédé de préparation d'une mousse de polyuréthane comprenant la réaction d'un polyisocyanate avec un matériau réactif avec un isocyanate en présence d'une composition azéotrope selon l'une des revendications 1 à 5.

7. Mousse de polyuréthane qui a été préparée en faisant réagir un polyisocyanate avec un matériau réactif avec un isocyanate en présence d'une composition azéotrope selon l'une des revendications 1 à 5.
